# EUROPEAN PATENT APPLICATION

(11) **EP 2 735 268 A1**
(43) Date of publication of application: **28.05.2014**
(21) Application number: 12306448.7
(22) Date of filing: 21.11.2012
(51) Int. Cl.: A61B 6/00, F16F 7/12, F16F 15/00, F16F 1/373, F16F 1/387

(54) **Anti-vibration holding system**

(71) Applicant: General Electric Company, Schenectady, New York 12345 (US)
(72) Inventor: Kamdoum Choumin, Hervé, 78533 Buc Cedex (FR)
(74) Representative: Hirsch & Associés

(57) **Abstract**

This invention relates to an anti-vibration holding system of a radiation source (1) on a C-arm (3) of a medical imaging system, comprising fasteners (6) fastening said radiation source (1) and said C-arm (3) together, vibration isolators (7) located between said radiation source (1) and said C-arm (3) to reduce vibration transmission from said radiation source (1) to said C-arm (3), wherein said vibration isolators (7) are respectively disposed around said fasteners (6), and wherein said fasteners (6) and said vibration isolators (7) are oriented, with respect to said radiation source (1) and to said C-arm (3), so that vibration transmission from said radiation source (1) to said C-arm (3) is reduced more by the radial stiffness of said vibration isolators (7) than by the axial stiffness of said vibration isolators (7).

## Description

### FIELD OF THE INVENTION

The invention relates to anti-vibration holding systems, and more particularly to anti-vibration holding systems of a radiation source on a C-arm of a medical imaging system.

### BACKGROUND OF THE INVENTION

No prior art is known to provide for an effective solution to the problem of either reducing the transmission of the radiation source vibrations to the rest of the medical imaging system or limiting the creation of vibrations in the radiation source.

According to a first potential solution internally tested by the Applicant, not to be considered as prior art, it was considered to reduce the transmission of the radiation source vibrations to the rest of the medical imaging system.

Indeed, in this first potential solution, fasteners fasten radiation source and C-arm together, vibration isolators are located between radiation source and C-arm to reduce vibration transmission from said radiation source to said C-arm, the vibration isolators being respectively disposed around the fasteners.

In this first potential solution, the fastening direction of the fasteners is oriented perpendicular to the mean plan of the plates to which the radiation source and the C-arm are respectively fixed. Then, any satisfactory compromise appears to be hard to get. Either the material of the vibrations isolators is soft enough to notably reduce the vibration transmission level between radiation source and C-arm, but it is then not stiff enough to provide acceptable image quality, the radiation source and the radiation detector being no more sufficiently aligned when the C-arm moves. Or the material of the vibration isolators is stiff enough to provide acceptable image quality, the radiation source and the radiation detector being sufficiently aligned when the C-arm moves, but it is then not soft enough to notably reduce the vibration transmission level between radiation source and C-arm.

According to the invention, it has been discovered that finding a compromise was too hard because of the perpendicularity between on the one hand the fastening direction of the fasteners and on the other hand the mean plan of the plates to which the radiation source and the C-arm are respectively fixed. Because of this perpendicularity, the fasteners and the vibration isolators are oriented, with respect to the radiation source and to the C-arm, so that vibration transmission from the radiation source to the C-arm is reduced more by the axial stiffness of said vibration isolators than by the radial stiffness of said vibration isolators.

By choosing to do the contrary, that is to say orienting the fasteners and the vibration isolators, with respect to the radiation source and to the C-arm, so that vibration transmission from the radiation source to the C-arm is reduced more by the radial stiffness of the vibration isolators than by the axial stiffness of the vibration isolators, the invention has solved the problem and has reached a good compromise allowing for simultaneous satisfactory reduction of vibration transmission between radiation source and C-arm on the one side, and satisfactory image quality due to the radiation source and the radiation detector being kept sufficiently aligned together when the C-arm moves, on the other side.

According to a second potential solution internally tested by the Applicant, not to be considered as prior art, it was considered to limit the creation of vibrations in the radiation source. Therefore, an active vibration control has been implemented within the radiation source. Vibrations propagation on the path between the vibrating anode of the radiation source and the casing of the radiation source has been limited. This second potential solution has proved complex to implement. This second potential solution is much more complex to implement than the solution in which the vibrations isolators of the invention are rightly oriented with respect to radiation source and C-arm.

None of the first and second potential solutions internally tested by the Applicant has yet proved satisfactory. Therefore, the invention proposes this new orientation of the vibrations isolators with respect to radiation source and C-arm, indeed contrary to the orientation of the vibrations isolators tested in the first potential solution.

### SUMMARY OF THE INVENTION

The object of the present invention is to alleviate at least partly the above mentioned drawbacks.

More particularly, the invention aims to reduce the vibration transmission between radiation source and C-arm, from radiation source to C-arm, by implementing vibration isolators between radiation source and C-arm which orientation is chosen to favor radial stiffness effect of the vibration isolators compared to axial stiffness effect of the vibration isolators. Preferably, the vibration isolators correctly oriented are located around the fasteners fastening radiation source and C-arm, because those fasteners are a weak point for vibrations transmission between radiation source and C-arm.

This object is achieved with an anti-vibration holding system of a radiation source on a C-arm of a medical imaging system, comprising fasteners fastening said radiation source and said C-arm together, vibration isolators located between said radiation source and said C-arm to reduce vibration transmission from said radiation source to said C-arm, wherein said vibration isolators are respectively disposed around said fasteners, and wherein said fasteners and said vibration isolators are oriented, with respect to said radiation source and to said C-arm, so that vibration transmission from said radiation source to said C-arm is reduced more by the radial stiffness of said vibration isolators than by the axial stiffness of said vibration isolators. Preferably, the medical imaging system also comprises a radiation detector which is aligned with said radiation source, and which should be kept aligned with said radiation source when the C-arm moves.

This object is also achieved with an anti-vibration holding system of a vibrating element on a mobile part of an apparatus, comprising fasteners fastening said vibrating element and said mobile part together, vibration isolators located between said vibrating element and said mobile part to reduce vibration transmission from said vibrating element to said mobile part, wherein said vibration isolators are respectively disposed around said fasteners, and wherein said fasteners and said vibration isolators are oriented, with respect to said vibrating element and to said mobile part, so that vibration transmission from said vibrating element to said mobile part is reduced more by the radial stiffness of said vibration isolators than by the axial stiffness of said vibration isolators. Preferably, when the mobile part moves, the amplitude of the displacements of the vibrating element with respect to the mobile part should be kept within a limited range. Said vibrating element is preferably a radiation source. Said radiation source is preferably an X-ray source belonging to a medical imaging system. Preferably, the medical imaging system also comprises a radiation detector which is aligned with said radiation source, and which should be kept aligned with said radiation source when the mobile part, preferably a C-arm, moves.

This object is still achieved with the anti-vibration holder located between the vibrating element, preferably a radiation source, and the mobile part of an apparatus, preferably a C-arm of a medical imaging system. This object is still achieved with an anti-vibration holder, comprising two plates parallel to each other, fasteners fastening one of said plates to the other of said plates along a fastening direction, vibration isolators located between said plates, wherein said vibration isolators are respectively disposed around said fasteners, and wherein said fastening direction of said fasteners is oriented parallel to the mean plan of said plates. When fastening means is for example a screw, the fastening direction is the axis of the screw.

Preferred embodiments comprise one or more of the following features, which can be taken separately or together, either in partial combination or in full combination.

Preferably, said fasteners and said vibration isolators are oriented, with respect to said radiation source and to said C-arm, so that vibration transmission from said radiation source to said C-arm is reduced essentially by the radial stiffness of said vibration isolators. All the more radial stiffness effect is favored compared to axial stiffness, the better it is to get at an easier compromise to implement between the vibration transmission reduction and the keeping of a good image quality.

Preferably, said radiation source is a tube emitting radiation and wherein said fasteners and said vibrations isolators are oriented parallel to said tube axis. This orientation allows for more efficiently favoring the radial stiffness effect compared to the axial stiffness effect. Preferably, said radiation source is a tube emitting radiation and wherein at least some of said fasteners and at least some of said vibrations isolators, preferably all of said fasteners and all of said vibrations isolators, each have an axis of symmetry which is oriented parallel to said tube axis.

Preferably, it also comprises a support located between said radiation source and said C-arm, and wherein said support supports said fasteners and said vibrations isolators. The presence of the intermediate element helps to localize the vibrations transmission more precisely and then to reduce this vibrations transmission more efficiently.

Preferably, said fasteners and said vibrations isolators are oriented parallel to the mean plan of said support. This orientation allows for more efficiently favoring the radial stiffness effect compared to the axial stiffness effect.

Preferably, said support is located between said C-arm and said vibration isolators. This is more efficient to reduce vibrations transmission than a location of this support only between said radiation source and said vibration isolators. Vibrations transmission is reduced before it can propagate too much within the system. Preferably, it comprises another support located between said radiation source and said vibration isolators.

Preferably, said support or each said support comprises flanges which are orthogonal to mean plan of said support and which comprise bores receiving said fasteners. The presence of the intermediate element helps to localize the vibrations transmission more precisely and then to reduce this vibrations transmission more efficiently. The narrowing of this intermediate element, the support or each support, helps to localize even more precisely the vibrations transmission path and to interact with it all the more efficiently.

Preferably, it comprises at least four of said vibration isolators, and preferably at most eight of said vibration isolators. A preferred minimum of four vibration isolators helps to increase stability of the whole system. A preferred maximum of eight vibration isolators helps not to increase too much system complexity.

Preferably, four of said vibration isolators are located at the four angles of a rectangle. This regular and spaced away configuration of the vibration isolators gives more efficiency to each vibration isolator.

Preferably, the distance between the two closest vibration isolators is more than 100mm, preferably more than 150mm, and wherein the distance between the two farthest vibration isolators is preferably less than 400mm, and more preferably less than 300mm.

Preferably, the radial stiffness of said isolators ranges from 150 to 500 daN/mm, and preferably ranges from 200 to 300 daN/mm. The combination between on the one side this material stiffness of the vibration isolators and the specific orientation of the vibration isolators helps achieve an optimized compromise between vibrations transmission reduction and image quality preservation.

Preferably, the ratio between a first distance and a second distance, first distance being between center of gravity of the whole group of said vibration isolators and center of gravity of the radiation source, second distance being between the two farthest vibration isolators, is less than 10%, and is preferably less than 5%. This location helps give a maximized effect to the vibration isolators on the vibrations transmission path.

Preferably, said fasteners are longer than they are large and said fasteners are preferably screws. This allows to better localize vibrations transmission path along these long screws and to better reduce vibrations transmission because of the vibration isolators surrounding these long screws. Any fastening device other than a screw, provided it is longer than large, would benefit of the same type of positive effect, at least in part.

Preferably, at least some of said vibration isolators, preferably all vibrations isolators, comprise, successively from center to periphery, a first metal tube located around a fastener, an elastomer tube located around said first metal tube, a second metal tube located around said elastomer tube. This vibration isolator structure in several concentric layers helps blocking more efficiently the vibrations transmission.

Preferably, said vibration isolators reduce said vibration transmission by at least 15dB, preferably by at least 20dB, more preferably by at least 25dB, in average over a vibration frequency range from 0 to 6kHz. Preferably, said vibration isolators reduce said vibration transmission by at most 50dB in average over a vibration frequency range from 0 to 6kHz. Preferably, said vibration isolators reduce the noise resulting from said vibration transmission by an amount ranging from 5 to 10dBA. Those reduction ranges are more than significant.

Further features and advantages of the invention will appear from the following description of embodiments of the invention, given as nonlimiting examples, with reference to the accompanying drawings listed hereunder.

### BRIEF DESCRIPTION OF THE DRAWINGS

Fig. 1 shows an exploded view of a part of a medical imaging system implementing an example of an anti-vibration holding system according to an embodiment of the invention;
Fig. 2 shows an exploded view of an example of a part of an anti-vibration holding system according to an embodiment of the invention;
Fig. 3 shows a view in perspective of an example of a part of an anti-vibration holding system according to an embodiment of the invention;
Fig. 4 shows a view in perspective of an example of the plate, close to the C-arm of a medical imaging system, of an anti-vibration holding system according to an embodiment of the invention;
Fig. 5 shows a view in perspective of an example of the plate, close to the radiation source of a medical imaging system, of an anti-vibration holding system according to an embodiment of the invention;
Fig. 6 shows curves showing the vibration reduction when using an example of an anti-vibration holding system according to an embodiment of the invention;
Fig. 7 shows curves showing the noise reduction when using an example of an anti-vibration holding system according to an embodiment of the invention.

### DETAILED DESCRIPTION OF THE INVENTION

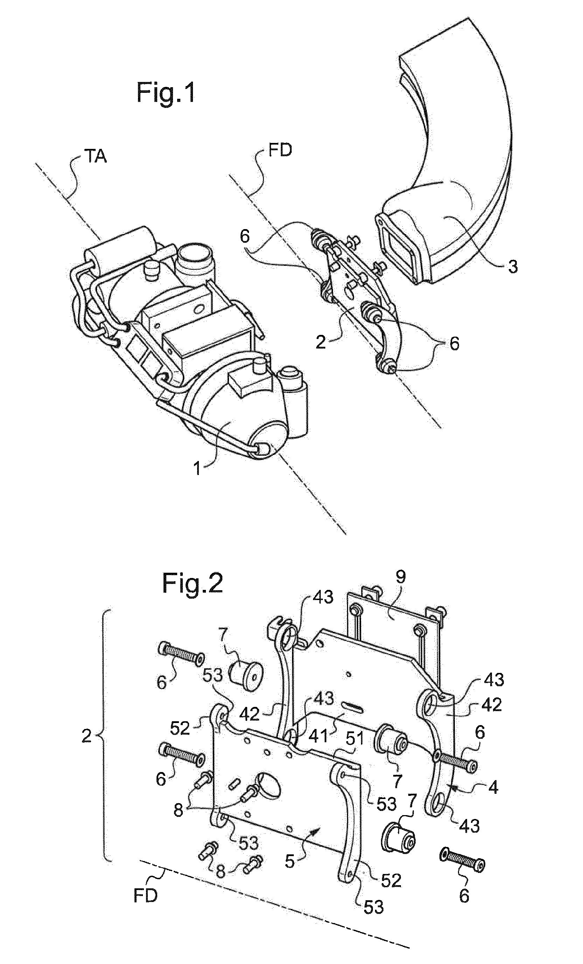

Fig. 1 shows an exploded view of a part of a medical imaging system implementing an example of an anti-vibration holding system according to an embodiment of the invention. A radiation source 1, preferably an X-ray tube which presents a tube axis TA, is fixed at the end of a C-arm 3, via an anti-vibration holder 2 which comprises screws 6 which present a fastening direction FD which is along the axis of the screws 6. Tube axis TA and fastening direction FD are parallel to each other.

Fig. 2 shows an exploded view of an example of a part of an anti-vibration holding system according to an embodiment of the invention. The anti-vibration holder 2 comprises a C-arm bracket 4 presenting the general form of a first plate 41 and a tube bracket 5 presenting the general form of a second plate 51, mean plan of this second plate 51 being parallel to the mean plan of the first plate 41.

The C-arm bracket 4 comprises flanges 42 on each side of the plate 41. Each flange 42 comprises bores 43, one bore 43 on each side of the flange 42. The tube bracket 5 comprises flanges 52 on each side of the plate 51. Each flange 52 comprises bores 53, one bore 53 on each side of the flange 52. Four screws 6 are respectively surrounded by four cylindrical vibration isolators 7. These screws 6 are respectively passing through both the bores 43 and the bores 53, so as to make plate 41 integral with plate 51. Supplementary screws 8 are used to attach tube 1 on tube bracket 5. A supplementary plate 9 is used to attach C-arm bracket 4 on C-arm 3. The fastening direction FD is parallel to the axis of the screws 6.

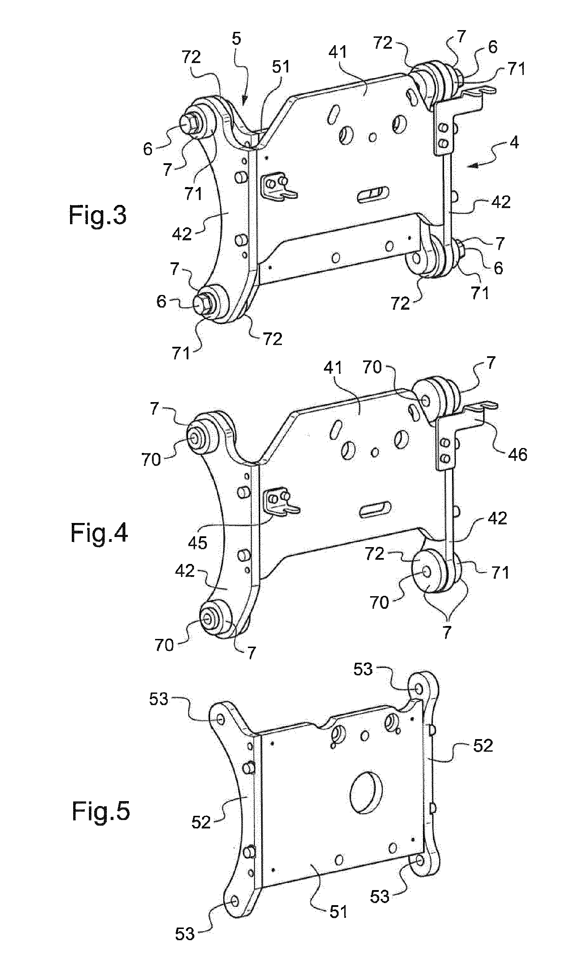

Fig. 3 shows a view in perspective of an example of a part of an anti-vibration holding system according to an embodiment of the invention. Tube bracket 5 and C-arm bracket 4 are attached together. Screws 6 extend through both flanges 42 and 52. Each vibration isolator 7 surrounds a screw 6. Each vibration isolator 7 extends further from flange 42 and presents a larger diameter between flange 42 and flange 52. Space between both flanges 42 and 52 is maintained to a nominal value approximately corresponding to the length of the larger part 72 of the vibration isolator 7. Along its length, the vibration isolator 7 presents on one side 72 a larger diameter than on the other side 71.

Fig. 4 shows a view in perspective of an example of the plate, close to the C-arm of a medical imaging system, of an anti-vibration holding system according to an embodiment of the invention. Each vibration isolator presents a bore 70 through which a screw 6 is intended to pass. On the side of the plate 41 which is opposite to the side from which the flanges 42 extend orthogonally, the plate 41 comprises two clamps 45 and 46, a clamp 45 in the form of a L, a clamp 46 in the form of a double L. Flanges 42 can be screwed on plate 41. Bores 43 are relatively large, since they are intended to receive both screws 6 and vibration isolators 7.

Fig. 5 shows a view in perspective of an example of the plate, close to the radiation source of a medical imaging system, of an anti-vibration holding system according to an embodiment of the invention. Plate 51 presents two flanges 52 which extend orthogonally on one side of plate 51. Bores 53 are smaller than bores 43 of plate 41, since they are intended to be passed through only by the screws 6 and not by the vibration isolators 7.

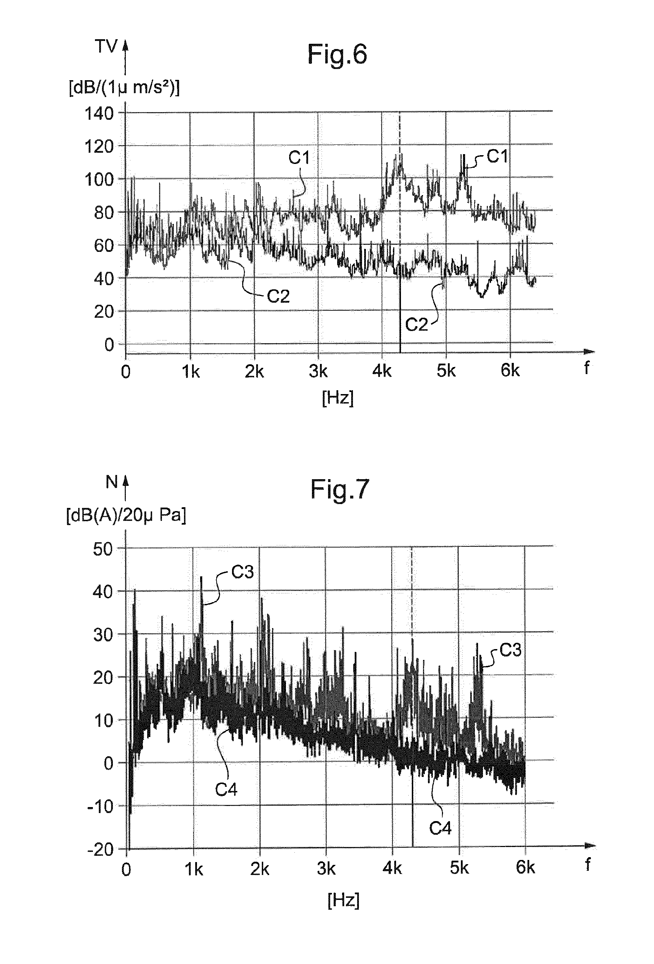

Fig. 6 shows curves showing the vibration reduction when using an example of an anti-vibration holding system according to an embodiment of the invention. The level of vibrations transmission TV from radiation source to C-arm, expressed in dB/(1µm/s²), is plotted versus the vibration frequency, expressed in Hz.

A first curve C1 represents the level of vibrations transmission TV as a function of the vibration frequency for a holding system without any vibration isolator.

A second curve C2 represents the level of vibrations transmission TV as a function of the vibration frequency for an anti-vibration holding system according to an embodiment of the invention. Over a vibration frequency spectrum ranging from 0kHz to 6kHz, the first curve C1 shows a variation of level of vibrations transmission TV which ranges from 60 to 120. Over a vibration frequency spectrum ranging from 0kHz to 6kHz, the second curve C2 shows a variation of level of vibrations transmission TV which ranges from 30 to 100 with an average value about 20% lower than the first curve C1. For the first curve C1, the RMS value is 130.2 dB/(1µm/s²), whereas for the second curve C2, the RMS value is 105.6 dB/(1µm/s²). So, about 25dB RMS (RMS = Root Mean Square, calculated on the whole frequency range of the signal) less of vibrations are transmitted from radiation source to C-arm in the second curve C2 as compared to the first curve C 1. Therefore, the reduction of transmission of vibrations from radiation source to C-arm can be considered as a substantial one. This reduction of transmission of vibrations is more important as it would be with an anti-vibration holding system according to said first potential solution discussed above.

Fig. 7 shows curves showing the noise reduction when using an example of an anti-vibration holding system according to an embodiment of the invention. The level of noise transmission N from radiation source to C-arm, expressed in dB(A)/20µPa, is plotted versus the vibration frequency, expressed in Hz.

A first curve C3 represents the level of noise transmission N as a function of the vibration frequency for a holding system without any vibration isolator.

A second curve C4 represents the level of noise transmission N as a function of the vibration frequency for an anti-vibration holding system according to an embodiment of the invention. Over a vibration frequency spectrum ranging from 0kHz to 6kHz, the first curve C3 shows a variation of level of noise transmission N which ranges from 0 to 45. Over a vibration frequency spectrum ranging from 0kHz to 6kHz, the second curve C4 shows a variation of level of noise transmission N which ranges from -10 to 35 with an average value about 10% lower than the first curve C3. For the first curve C3, the RMS value is 55.5 dB(A)/(20µPa), whereas for the second curve C4, the RMS value is 49.7 dB/(20µPa). So, about 5.5dBA RMS (RMS = Root Mean Square, calculated on the whole frequency range of the signal) less of noise is transmitted from radiation source to C-arm in the second curve C4 as compared to the first curve C3. Therefore, the reduction of transmission of noise from radiation source to C-arm can be considered as a substantial one too. This reduction of transmission of noise is more important as it would be with an anti-vibration holding system according to said first potential solution discussed above.

The invention has been described with reference to preferred embodiments. However, many variations are possible within the scope of the invention.

## Claims

1. Anti-vibration holding system of a radiation source (1) on a C-arm (3) of a medical imaging system, comprising :
- fasteners (6) fastening said radiation source (1) and said C-arm (3) together,
- vibration isolators (7) located between said radiation source (1) and said C-arm (3) to reduce vibration transmission from said radiation source (1) to said C-arm (3),
wherein said vibration isolators (7) are respectively disposed around said fasteners (6),
and wherein said fasteners (6) and said vibration isolators (7) are oriented, with respect to said radiation source (1) and to said C-arm (3), so that vibration transmission from said radiation source (1) to said C-arm (3) is reduced more by the radial stiffness of said vibration isolators (7) than by the axial stiffness of said vibration isolators (7).

2. Anti-vibration holding system according to claim 1, wherein said fasteners (6) and said vibration isolators (7) are oriented, with respect to said radiation source (1) and to said C-arm (3), so that vibration transmission from said radiation source (1) to said C-arm (3) is reduced essentially by the radial stiffness of said vibration isolators (7).

3. Anti-vibration holding system according to any of preceding claims, wherein said radiation source (1) is a tube emitting radiation and wherein said fasteners (6) and said vibrations isolators (7) are oriented parallel to said tube axis (TA).

4. Anti-vibration holding system according to any of preceding claims, wherein said radiation source (1) is a tube emitting radiation and wherein at least some of said fasteners (6) and at least some of said vibrations isolators (7), preferably all of said fasteners (6) and all of said vibrations isolators (7), each have an axis of symmetry which is oriented parallel to said tube axis (TA).

5. Anti-vibration holding system according to any of preceding claims, wherein it also comprises a support (4, 5) located between said radiation source (1) and said C-arm (3), and wherein said support (4, 5) supports said fasteners (6) and said vibrations isolators (7).

6. Anti-vibration holding system according to claim 5, wherein said fasteners (6) and said vibrations isolators (7) are oriented parallel to the mean plan of said support (4, 5).

7. Anti-vibration holding system according to any of claims 5 to 6, wherein said support (4) is located between said C-arm (3) and said vibration isolators (7).

8. Anti-vibration holding system according to claim 7, wherein it comprises another support (5) located between said radiation source (1) and said vibration isolators (7).

9. Anti-vibration holding system according to any of claims 5 to 8, wherein said support (4, 5) or each said support (4, 5) comprises flanges (42, 52) which are orthogonal to mean plan of said support (4, 5) and which comprise bores (43, 53) receiving said fasteners (6).

10. Anti-vibration holding system according to any of preceding claims, wherein it comprises at least four of said vibration isolators (7), and preferably at most eight of said vibration isolators (7).

11. Anti-vibration holding system according to claim 10 wherein four of said vibration isolators (7) are located at the four angles of a rectangle.

12. Anti-vibration holding system according to any of preceding claims, wherein the distance between the two closest vibration isolators (7) is more than 100mm, preferably more than 150mm, and wherein the distance between the two farthest vibration isolators (7) is preferably less than 400mm, and more preferably less than 300mm.

13. Anti-vibration holding system according to any of preceding claims, wherein the radial stiffness of said isolators ranges from 150 to 500 daN/mm, and preferably ranges from 200 to 300 daN/mm.

14. Anti-vibration holding system according to any of preceding claims, wherein the ratio between a first distance and a second distance, first distance being between center of gravity of the whole group of said vibration isolators (7) and center of gravity of the radiation source (1), second distance being between the two farthest vibration isolators (7), is less than 10%, and is preferably less than 5%.

15. Anti-vibration holding system according to any of preceding claims, wherein said fasteners (6) are longer than they are large and wherein said fasteners (6) are preferably screws.

16. Anti-vibration holding system according to any of preceding claims, wherein at least some of said vibration isolators (7), preferably all vibrations isolators (7), comprise, successively from center to periphery, a first metal tube located around a fastener (6), an elastomer tube located around said first metal tube, a second metal tube located around said elastomer tube.

17. Anti-vibration holding system according to any of preceding claims, wherein said vibration isolators (7) reduce said vibration transmission by at least 15dB, preferably by at least 20dB, more preferably by at least 25dB, in average over a vibration frequency range from 0 to 6kHz, and wherein said vibration isolators (7) preferably reduce said vibration transmission by at most 50dB in average over a vibration frequency range from 0 to 6kHz.

18. Anti-vibration holding system according to any of preceding claims, wherein said vibration isolators (7) reduce the noise resulting from said vibration transmission by an amount ranging from 5 to 10dBA.

19. Anti-vibration holding system of a vibrating element (1) on a mobile part (3) of an apparatus, comprising :
- fasteners (6) fastening said vibrating element (1) and said mobile part (3) together,
- vibration isolators (7) located between said vibrating element (1) and said mobile part (3) to reduce vibration transmission from said vibrating element (1) to said mobile part (3),
wherein said vibration isolators (7) are respectively disposed around said fasteners (6),
and wherein said fasteners (6) and said vibration isolators (7) are oriented, with respect to said vibrating element (1) and to said mobile part (3), so that vibration transmission from said vibrating element (1) to said mobile part (3) is reduced more by the radial stiffness of said vibration isolators (7) than by the axial stiffness of said vibration isolators (7).

20. Anti-vibration holder, comprising :
- two plates (41, 51) parallel to each other,
- fasteners (6) fastening one of said plates (41, 51) to the other of said plates (41, 51) along a fastening direction (FD),
- vibration isolators (7) located between said plates (41, 51),
wherein said vibration isolators (7) are respectively disposed around said fasteners (6),
and wherein said fastening direction (FD) of said fasteners (6) is oriented parallel to the mean plan of said plates (41, 51).
